# EUROPEAN PATENT SPECIFICATION

(11) **EP 3 622 948 B1**
(45) Date of publication and mention of the grant of the patent: **06.05.2026**
(21) Application number: 19195219.1
(22) Date of filing: 03.09.2019
(51) Int. Cl.: A61K 9/16

(54) **MULTILAYERED FORMULATIONS WITH DUAL RELEASE RATE OF ONE OR MORE ACTIVE PRINCIPLES**
MEHRSCHICHTIGE FORMULIERUNGEN MIT DUALER FREISETZUNGSRATE EINES ODER MEHRERER WIRKSTOFFE
FORMULATIONS MULTICOUCHES À DOUBLE DÉBIT DE LIBÉRATION D'UN OU PLUSIEURS PRINCIPES ACTIFS

(30) Priority: 11.09.2018 IT 201800008513
(43) Date of publication of application: 18.03.2020
(73) Proprietor: I.P.S. International Products & Services S.r.l., 20097 S. Donato Milanese (IT)
(72) Inventor: PASOTTI, Gino, 20097 SAN DONATO MILANESE (IT); SPALLA, Andrea, 20097 SAN DONATO MILANESE (IT); PENGO, Sergio, 20097 SAN DONATO MILANESE (IT); SPALLA, Luciano, 20097 SAN DONATO MILANESE (IT)
(74) Representative: Riccardi, Sergio

(56) References cited:
- WO-A1-2009/033072
- US-A1- 2008 254 121
- US-A1- 2008 299 188
- US-A1- 2011 033 506
- US-A1- 2015 290 147
- US-A1- 2016 199 378

## Description

### FIELD OF THE INVENTION

The present invention relates to multilayered miniparticles having a particle size ranging from 200 µm to 850 µm for oral use which allow both the slower release and the faster release of one or more active principles or active substances or matrices containing them, to a process for their preparation and to specific forms of administration for oral use containing them.

### STATE OF THE ART

For a long time in the prior art modified release systems have been known constituted by a core comprising an inert material that is coated with a layer of the active ingredient or the active substance in turn covered with a layer of modified release membrane able to release the active ingredient/active substance to the stomach/small intestine or colon depending on the characteristics of the layer of the membrane used.

Systems are also known as the Applicant's proprietory technology MATRIS^{®} (Multiform Administration Timed Release Ingredients System, i.e. single matrix for the administration of active ingredients having a modified release and in different oral forms), for the controlled release of principles or active substances, without the use of neutral materials/inert materials. In this way, the concentration of the active ingredient/active substance in final form is considerably higher than the modified release forms known in the art. Said technology provides for covering each particle of the active ingredient with a suitable modified release membrane. The technology MATRIS^{®} allows the controlled release of the active principle or active substance further to the total coverage of unpleasant tastes of the active ingredients and to provide any form of administration for oral use, such as, in additions to high concentration capsules, monodose sachets and tablets, also orosoluble, extemporaneous suspensions and monodose vials.

MATRIS^{®} overcomes the obstacle of the limited possibility of unitary administration of the active ingredients, due to the poor capacity of the capsule, particularly relevant problem with food supplements, whose dosage is very often higher than the one of drugs. MATRIS^{®} also overcomes the problem of swallowing capsules for a better acceptance or *compliance* of the final user, especially in case of children and older people. Said technology also overcomes the disadvantages of the pellets that generally allow the release of limited amounts of active principle or substance since approximately 50% of their content is constituted by inert substances such as sugar and starch.

In more recent times, however, there has been highlighted the problem of the need to provide a formulation having the same active ingredient or substance both with slow release and with fast release (so as to obtain for example a finished product with both a fast and a retard release of the active ingredient(s)). This is because for the treatment of specific diseases it is required a daily intake of two or more active substances in combination, or of the same active substance with different time releases or in different amounts. Therefore, to comply with this requirement in pharmaceutical technology and in the technology of supplements, individual forms for oral administration were made having a different content of active ingredient or dosage units also comprising two or more release forms even of different active principles.

For example, compositions are known which comprise as dosage units a slow or controlled release formulation and another fast or immediate release formulation of topiramate and phentermine respectively (see for example US 2017/151179), in which the amounts of each active ingredient in the dosage form are chosen independently from one another and are specific for each active ingredient.

In this regard, a tablet consisting of two parts having different active ingredients and different kinds of delivery (i.e. fast and slow), such as for example phentermine and topiramate, is also described in CN 106880640.

Another example of tableted formulations, particulary for cardiovascular health (e.g., niacin and atorvastatin) are described in patent application WO 2009/033072 A1.

In any case, these specific pharmaceutical forms tend to be rather voluminous in so far as they provide for the presence of the principles or active substances in combination with more excipients, thus said pharmaceutical forms are hardly swallowable or require to be divided and this has a negative impact on the administration and acceptance of these forms of administration by the users, as well as on the release times of the various active principles.

There is also the disadvantage linked to the fact that the entire dosage unit after ingestion is located in only one point of the gastrointestinal tract, where the concentration of the released active principle/principles is very high, with risk of local irritations. In addition, it is possible that the entire dose ingested is arranged, during the journey in the gastrointestinal tract, in a position not so favorable and/or at a time not proper for the absorption, with loss of substance and then of a part of the active principles.

These drawbacks are clearly avoided with the administration of active principle/principles divided into tiny mini-doses, which spread uniformly throughout the gastrointestinal tract, thus avoiding a high concentration in a defined point and reducing the probability of a failure to release a single dose at a specific location or at a time not very favorable for the absorption, at the expense of bioavailability.

In particular, the embodiments of the known art US 2016/199378, WO 2009/033072, US 2015/290147, US 2011/033506 and US 2008/299188 the entire dose of active principle/active substance is enclosed in a single solid form of administration which releases its content in accordance with predetermined procedures.

Moreover, the dosage forms of the prior art are rather laborious to prepare and are not very suitable for compression or heat sensitive active principles or substances.

There is therefore the need to provide a formulation for oral use which overcomes all the disadvantages of the formulations of the prior art and in particular which is easily preparable and administrable, with the satisfaction of the final user and which allows a release and absorption profile which can be controlled and then profiles of reproducible bioavailability.

### Summary of the invention

The present invention is a multilayered miniparticle having a particle size ranging from 200 µm to 850 µm, for oral administration comprising from the inside to outside:
A) a core of an active substance;
B) an inner film layer which is protective or for the modified release;
C) a layer of at least an active substance which is equal to or different from the one in the core A);
D) an outer film layer which more rapidly releases compared to the inner film layer which is protective or for the modified release B), wherein the active substance is selected in the group comprising thioctic or alpha lipoic acid, iron or a salt thereof such as ferrous fumarate or bisglycinate, vitamin C, folic acid.

It is an object of the present invention a process for the preparing a multilayered miniparticle having a particle size ranging from 200 µm to 850 µm, as defined above comprising the following steps:
i. sieving and sorting an active substance having varied particle size to obtain particles of active substance with defined particle size;
ii. applying a solution of a polymer or film which is protective or for the modified release to each particle of active substance of step i. to obtain a core of active substance covered with a film layer which is protective or for the modified release;
iii. forming a layer of at least an active substance which is equal to or different from the one present in the core, on the covered core of active substance of step ii.;
iv. forming, on the layer formed at step iii, an outer film which releases more rapidly compared to the film layer which is protective or for the modified release of step ii.

It is a further object of the present invention a matrix, optionally a polymer, or of other type suitable for use in the field of supplements or pharmaceuticals, comprising miniparticles as previously defined.

The technology of the present invention, defined by the denomination M.A.T.R.I.S. DUAL , is an innovative solution, based on the problem-solution approach, allowing to combine multiple active ingredients within a single delivery unit of multi-layered miniparticles, having a particle size of 200 - 850 microns, preserving the capabilities of controlled release and flavour and odor masking. This allows to solve, in the steps of industrial production of the final administration forms, the problems due to blend loss, namely the heterogeneous lamination of active ingredients having different densities. Moreover, the transit in the gastro-enteric tract of different active ingredients within a single above mentioned miniparticle delivery unit, prevents the separated localization and/or segregation of these active ingredients with the consequent optimization of the bioavailability. Finally, since the volume occupied by one single delivery unit of miniparticles, is lower than the sum of the volumes occupied by single active ingredient in the same form, the here described technology allows to make higher unitary dosages, thus contributing, together with the higher bioavailability, to reduce the frequency administration (better patient compliance).

It is a further object of the present invention a formulation for oral use selected in the group comprising a sachet, a tablet, a capsule, a granulate, a vial, an extemporaneous suspension that contains a set of more multilayered miniparticles as defined above and prepared with the processes listed above.

### Brief description of figures

Figure 1 discloses an example of regular miniparticle X and an example of irregular miniparticle Y with dual release mechanism.
Figure 2 discloses miniparticles X and/or Y in a matrix that acts as a container for the release of the same miniparticles.

### Detailed description of the invention

The invention therefore relates to a multilayered miniparticle having a particle size ranging from 200 µm to 850 µm, for oral administration comprising from inside to outside:
A) a core of an active substance;
B) an inner film layer which is protective or for the modified release;
C) a layer of at least an active substance which is equal to or different from the one in the core A);
D) an outer film layer which more rapidly releases compared to the inner film layer which is protective or for the modified release B), wherein the active substance is selected in the group comprising thioctic or alpha lipoic acid, iron or a salt thereof such as ferrous fumarate or bisglycinate, vitamin C, folic acid.

In accordance with the present invention the multilayered miniparticle as claimed for oral administration can be advantageously used both in the context of the supplements that in the pharmaceutical field.

Surprisingly, in this form of administration which has a "dual" release mechanism the single miniparticles of one or more active substances depending on the active substances considered are the ones to be coated directly and individually by layers of different membranes or by a single membrane in different amounts, thus obtaining the release of the single miniparticles of active substance/active substances according to the defined mode.

The set of miniparticles can then be enclosed in a matrix that is nothing more than a simple container/dispenser intended, immediately after ingestion, to promptly release all the miniparticles.

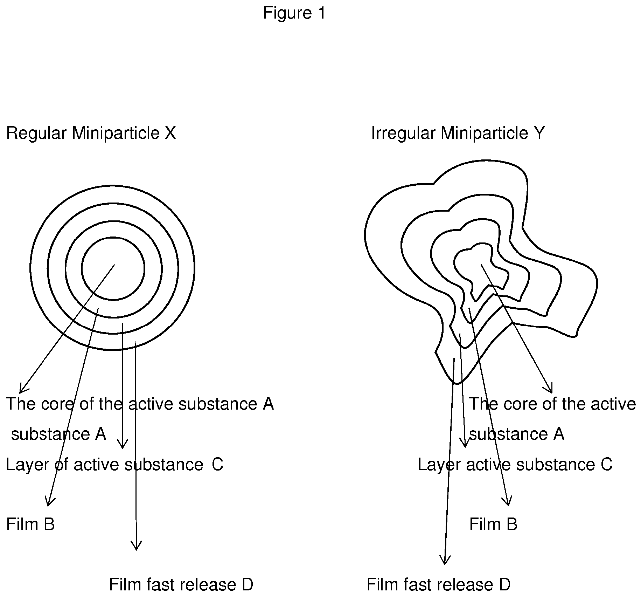

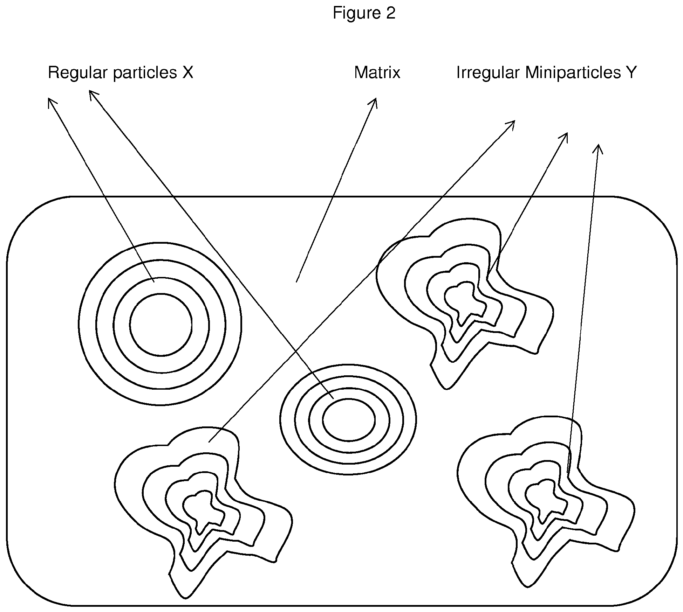

In this connection see Figures 1 and 2.

This dosage form thereby allows the active substance/active substances to quickly reach, after administration, the whole gastrointestinal tract, initiating the modified release at the level of the stomach or intestine, according to the properties of the membranes used.

And this is also in consideration of the particular characteristics of fineness and smoothness of the miniparticles, which allow the active substance/active substances to spread so fastly and uniformly over the entire surface of the gastrointestinal tract.

Said miniparticle, with regular or irregular shape, in accordance with the present invention is not in any way suggested by particles described in the prior art.

Typically, the miniparticle of the present invention has various, for example rounded shape and a regular or irregular surface and the multilayered structure has concentric layers.

More miniparticles together constitute a veritable small granulate that can be used as a basic product for the implementation of the final forms of administration that can be placed on the market.

The core of an active substance A), as defined above, can have a diameter comprised ranging from 200 µm to 850 µm and is preferably 188 µm-630 µm in case the active substance is thioctic acid or is 250 µm-790 µm in case the active substance is caffeine.

The term " active substance" in accordance with the present invention it is referred to a substance selected from the group comprising thioctic acid or alpha lipoic acid, iron or a salt thereof such as ferrous fumarate or bisglycinate, vitamin C, folic acid,.

The inner layer of the protective or modified release film (B) may typically be with extended or delayed release and may be made of a substance or a polymer selected from the group comprising ethylcellulose, hydroxypropylmethylethylcellulose phthalate(HPMCP), hydroxypropylmethylethylcellullose acetate succinate (HPMCAS), ethylene-vinyl acetate, a derivative of the polyacrylic acid, acrylic derivatives: copolymers of acrylic acid/methylmethacrylate (i.e. EUDRAGIT), shellac, hydroxypropylmethylcellulose (HPMC) and hydroxypropylcellulose (HPC).

Preferably, the inner layer (B) is made of a slow release (retard) film, where the release of the active substance occurs in a time equal to or higher than 3600 s (hours) .

Preferably, the inner layer of the film (B) consist of ethyl cellulose or hydroxypropyl cellulose (HPC) and optionally shellac.

Preferably, the inner layer B) can be obtained from a solution of ethylcellulose (for example ethylcellulose in ethanol) with addition of talc or starch, preferably talc.

More preferably, the inner layer B) is formed from a solution of 5% w/w ethylcellulose in 96 % ethanol with the possible addition of talc.

Alternatively, in another preferred aspect the inner layer B) can be obtained starting from a solution with shellac and 20% w/w hydroxypropylcellulose in 96% ethanol with the possible addition of talc.

The inner layer B can have a release rate equal to 10%-50% of the release rate of the layer D.

The layer of active substance C) may be formed by an active substance equal to or different from those used for the core as defined above and may contain shellac or other binding polymer.

In a preferred aspect of the invention the layer C) is made of thioctic acid as the core A) of the miniparticle or the layer C) is made of caffeine as the core A) of the miniparticle.

Alternatively in another preferred aspect of the invention the layer C) is made of acetyl L-carnitine hydrochloride and the core A) of thioctic acid.

In the two types of technical solution it is possible to obtain the release of the same active substance with two different profiles (dual release fast plus slow) or two release profiles of two different active substances such as thioctic acid and acetyl L-carnitine hydrochloride.

The layer of active substance C) can also contain shellac, polyvinylpyrrolidone (PVP) or HPC (i.e. Klucel).

The layer of active substance C) has a thickness equal to or less than 200 µm and is typically comprised between 1 and 200 µm for any active substance used in layer C).

The outer layer (D) can be made of a substance or polymer selected from the group comprising PCL-PVAc-PEG (i.e Soluplus^{®),} hydroxypropylmethylcellulose (HPMC), polyethylene glycol (PEG), hydroxyethyl cellulose (HEC), hydroxypropyl cellulose (HPC), polyvinyl alcohol (PVA), polyvinylpyrrolidone (PVP), shellac, ethylcellulose and acrylic derivatives: copolymers of acrylic acid/methylmethacrylate (i.e. EUDRAGIT).

Preferably, in the case of miniparticle that contains only thioctic acid, the outer layer (D) is made of ethylcellulose and can be obtained starting from a solution of ethylcellulose (for example 5% w/w ethylcellulose in ethanol) with the possible addition of talc.

Not claimed, in the case of a miniparticle that contains only caffeine, the outer layer (D) is made of hydroxypropylcellulose and shellac and can be obtained starting from a solution of shellac and 20% w/w hydroxypropylcellulose with the possible addition of talc.

If the outer layer (D) is made of the same polymer or substance of the inner layer (B), the slower release of the active substance present in the core can be obtained in such a way that the inner layer (B) has a thickness greater than that of the outer layer (D).

Typically, the outer layer (D) not only releases more rapidly with respect to the layer of inner film or modified release film (B), but can also have simultaneously or alternately an effect of masking the taste (*taste-masking effect*). The rate of release of the layer D is typically comprised between 50 - 90 %, and is therefore greater than that of the inner layer (B).

The outer layer D) if made with hydroxypropyl methylcellulose (HPMC) has a marked effect of masking the taste.

The inner layer B) or outer layer (D) if made with methacrylic acid allows to adjust the release (immediate, enteric or in the colon).

In accordance with a particularly preferred aspect of the invention the active substance present in the miniparticle is thioctic acid only where the thioctic acid is released from the respective miniparticle firstly in a faster way and then in a slower way (release profile FAST + RETARD).

Alternatively, not claimed, two active substances may be used in the miniparticle i.e. thioctic acid and acetyl L-carnitine hydrochloride.

In this case the thioctic acid is released more slowly while the acetyl L-carnitine hydrochloride in a faster way.

It is noted that the *particle size* of the final miniparticle is comprised between about 200 µm and about 850 µm, preferably between 210 µm and 850 µm.

As already highlighted, the miniparticles of the present invention in particular having a *particle size* of 210-850 µm have the shape of the small granulate that can be used as such or for the preparation of other forms of administration intended for marketing.

Said granulate may be especially directly put in sachets or inserted into capsules or compressed to obtain tablets, also rapid disintegration tablets (ODTs). The granulate can also be used for the preparation of extemporaneous suspensions or inserted in monodose vials.

It is an object of the present invention a process for the preparation of a miniparticle as claimed above comprising the following steps:
i. sieving and sorting an active substance having varied particle size to obtain particles of active substance with defined particle size;
ii. applying a solution of a polymer or film which is protective or for the modified release to each particle of active substance of step i. to obtain a core of active substance covered with a film layer which is protective or for the modified release;
iii. forming a layer of at least an active substance which is equal to or different from the one present in the core, on the covered core of active substance of step ii.;
iv. forming, on the layer formed at step iii, an outer film which releases more rapidly compared to the film layer which is protective or for the modified release of step ii.

With *particle size* is meant the granulometry.

With varied *particle size* with reference to the step i. refers to particles having a not defined *particle size.*

With *particle size* defined in accordance to step i. is meant a *particle* size comprised in particular between 150 µm and 800 µm, more preferably between 188 µm and 630 µm in case of particles of thioctic acid or between 250 µm and 790 µm in case of particles of caffeine.

The step i. of the process is carried out using sieves or vibrating sieves with appropriate meshes that allow the passage of the particles of the active substance of suitable size (for example vibrating screens with net from 790, 630, 250 and 188 µm).

The solution of a polymer or protective film or for release modified in step ii. is a polymer or film which forms the inner layer B) of the miniparticle, as defined above and said solution is prepared according to known methods.

The steps ii-iii-iv of this preparative process can be carried out according to the methods of pharmaceutical technology or of the supplements.

The solution of a polymer or film to step ii) may for example be made starting from a solution of 5% w/w ethylcellulose in 96% ethanol with the possible addition of talc or shellac and 20% w/w hydroxypropylcellulose in 96% ethanol with the possible addition of talc.

The active substance present in the core and in the layer in step iii. can be thioctic acid; alternatively but not claimed the active substance present in the core and in the layer in step iii. can be caffeine.

In another not claimed aspect of the invention the active substance present in the core is thioctic acid and the active substance to step iii. is acetyl L-carnitine hydrochloride.

The layer of active substance to step iii, typically having a thickness equal to or less than 200 µm, preferably between 1µm and 200 µm, can be placed on the layer ii, for example using a solution of a substance or polymer, such as shellac, or other binding polymers.

The formation of the outer layer of the film at step iv. is the preparation of the outer layer of the film D) of the multilayered miniparticle as disclosed above.

The formation of the outer layer of the film at step iv. can be obtained starting from a solution of 5% w/w ethylcellulose in 96 %ethanol with the possible addition of talc or shellac and 20% w/w hydroxypropylcellulose in 96% ethanol with the possible addition of talc.

The multilayered miniparticle as obtained by the process of the present invention has a defined *particle size:* for example the multilayered miniparticle with thioctic acid with a faster and a slower release has a *particle size* preferably comprised between 210 µm and 850 µm therefore after the step iv. of the above mentioned process typically a sieving and particle size selection are made of the miniparticles obtained in step iv. similar to that of step i. of the process of the present invention (i.e. selection of *particle size* of multilayered miniparticles obtained in step iv. with appropriate nets for sieves or vibrating sieves), so as to obtain the multilayered miniparticles with the desired *particle* size, namely ranging from 200 µm to 850 µm.

Steps of sieving and sorting only partially coated miniparticles may also be present after some steps of the process disclosed above, for example after the step ii. and/or after step iii. Said sieving steps are similar to step i. of the process disclosed above, possibly changing only the size of the net of sieves/vibrating sieves used.

In a preferred aspect of the invention, in the process as disclosed above after each of the steps ii., and/or iii and/or iv. there is an additional step of sieving and sorting the material obtained, optionally carried out with appropriate sieves/vibrating sieves with nets of suitable dimensions.

It should be noted that the type of film used to make the layer adhering to the core (step ii), and the outer layer (step iv.) can influence in a decisive way where the active substance will be released and then absorbed.

In particular, the miniparticles with thioctic acid with slower and faster release disclosed in the present patent application can have both the inner layer (B) and the outer layer (D) of ethyl cellulose or hydroxypropyl cellulose and possibly shellac.

It is obtained a dual release profile of the thioctic acid since the two films of ethylcellulose (outer layer and inner layer) have different thicknesses and then release the active substance in different times, as well as highlighted in examples (see example 1).

Moreover, it is obtained a dual release profile of caffeine because the two films of hydroxypropylcellulose and shellac (outer layer and inner layer) have different thicknesses and then release the active substance in different times, as well as highlighted in the examples (see example 2).

It is a further object of the present invention as claimed, is a multilayered miniparticle for oral administration comprising from inside to outside:
a) a core of an active substance;
b) an inner film layer which is protective or for the modified release;
c) a layer of at least an active substance equal to or different from the one of the core a);
d) a layer of at least an active substance equal to or different from the ones of the core a) or of the layer c);
e) an outer film layer which releases more rapidly than the inner film layer which is protective or for the modified release b).

This miniparticle has characteristics similar to the miniparticle with dual release mechanism as described above.

In the present case with "active substance" it is meant an active substance wherein the active substance is selected in the group comprising thioctic or alpha lipoic acid, iron or a salt thereof such as ferrous fumarate or bisglycinate, vitamin C, folic acid. In particular, this latter multilayered miniparticle allows the progressive release of three different active substances such as vitamin C, folic acid and ferrous fumarate or bisglycinate.

In fact, it has been surprisingly found that a multilayered miniparticle as the latter just defined (with core a) and layers b) and e)) is particularly advantageous especially for administration and absorption of iron.

In agreement with the latter aspect of the invention it is possible for example administering vitamin C, folic acid and ferrous fumarate or bisglycinate obtaining a progressive release of said active substances.

As is known, vitamin C promotes the absorption of iron.

Folic acid is essential for the synthetic reactions involved in the formation of red blood cells and in the methylation of DNA.

The miniparticle with vitamin C, folic acid and ferrous fumarate or bisglycinate according to the present invention typically being orodispersible or orosoluble allows an easier intake (greater satisfaction of the final user), and is especially suitable for administration to older people.

In a preferred aspect of the invention the core a) and the layers (c) and (d) are made each of a different active substance.

More preferably the core (a) of the latter miniparticle is made of vitamin C, the layer c) is made of folic acid and the layer d) is made of iron or a salt thereof such as for example ferrous fumarate or bisglycinate.

Furthermore, the inner layer of protective film or for the modified release (b) can be made of a substance or a polymer selected from the group comprising ethylcellulose, hydroxypropylmethyethylcellulose phthalate (HPMCP), hydroxypropylmethyethylcellulose acetate succinate (HPMCAS), ethylene vinyl acetate, a derivative of the polyacrylic acid, acrylic derivatives: copolymers of acrylic acid/methylmethacrylate (i.e. EUDRAGIT), shellac, hydroxypropylmethylcellulose (HPMC) and hydroxypropylcellulose (HPC), preferably in HPMC.

Preferably, the inner layer b) can be obtained starting from a hydroalcoholic solution of 5% w/w HPMC in 96% ethanol/filtered H₂0 and with the possible addition of talc.

The layer c) may contain in addition to iron or a salt thereof, such as ferrous fumarate or bisglycinate, shellac or another binding polymer.

The outer layer of protective film or for the modified release e) is made of a substance or polymer selected from the group comprising PCL-PVAc-PEG (Soluplus^{®}), hydroxypropylmethylcellulose (HPMC), polyethylene glycol (PEG), hydroxyethyl cellulose (HEC), hydroxypropyl cellulose (HPC), polyvinyl alcohol (PVA), polyvinylpyrrolidone (PVP), shellac, ethylcellulose and acrylic derivatives: copolymers of acrylic acid/methylmethacrylate (i.e. EUDRAGIT) preferably in shellac and hydroxypropylcellulose, the outer layer of protective film e) may also have an effect of masking the taste *(taste-masking* effect).

Preferably the outer layer e) is prepared from a solution of shellac and 20%w/w hydroxypropylcellulose in 96% ethanol and with the possible addition of talc.

The core of active substance a) has a diameter typically comprised between 150 µm and 800 µm, preferably between 250 and 630 µm.

The layers (c) and (d) have a thickness typically less than or equal to 200 µm, preferably between 1 and 200 µm.

The layers (b) and (e) have a thickness typically less than or equal to 200 µm, preferably between 1 and 200 µm, and a release rate of the layer e) is equal to 50 - 90% of the release rate of the layer b).

Even the latter multilayered miniparticle has a structure of typically concentric layers.

Said latter multilayered miniparticle have a *particle size* ranging typically between about 200 and about 850 µm, preferably between 250 µm and 790 µm.

It is a further object of the present invention a process for the preparing a miniparticle as just disclosed above comprising the following steps:
I. sieving and sorting an active substance having a varied particle size to obtain particles of active substance having defined particle size;
II. applying a solution of a polymer or film which is protective or for the modified release to each particle of active substance of step I. to obtain a core of active substance covered with a film layer which is protective or for the modified release;
III. forming a layer of at least an active substance, equal to or different from the one of the core, on the covered core of step II;
IV. forming a layer of at least an active substance on the layer formed at step III.;
V. forming an outer film layer, on the layer formed at step IV, which releases more rapidly compared to the film layer which is protective or for the modified release of step II.

With varied *particle size* in reference to step I. it is meant particles of the active substance of not defined size.

With *particle size* defined in accordance to step I. it is meant a *particle* size ranging typically between 200 µm and 800 µm, preferably between 250 and 630 µm.

The step I. of the process is performed using sieves or vibrating sieves with appropriate mesh that allows the passage of the particles of the active substance of suitable size (for example vibrating sieves with net of 630 µm and 250 µm).

The active substance of step I. is typically vitamin C (also herein defined as Vit. C).

A solution of a polymer or protective substance or for the modified release at step II is a polymer or substance that makes the inner layer b) of the miniparticle, as defined above, and the solution is prepared according to known methods.

Typically the preparation of the polymer solution or film according to step II. can be carried out using 5% w/w HPMC in 96% ethanol/ filtered H₂O and with the possible addition of talc.

The steps II, III, IV, V of this preparative process can be carried out according to the methods of pharmaceutical technology or of the supplements.

The layer of active substance at step III, typically having a thickness equal to or less than 200 µm, preferably between 1 and 200 µm, is of folic acid.

The layer of active substance of step IV, typically having a thickness equal to or less than 200 µm, preferably between 1 and 200 µm, is typically of ferrous fumarate or bisglycinate and may also contain shellac or other binding polymer.

The outer layer of the film at step V can be made starting from a solution of shellac and 20% w/w hydroxypropylcellulose in 96 % ethanol with the possible addition of talc.

The multilayered miniparticle as obtained according to the process just disclosed above has a *particle size* defined typically between 250 µm and 790 µm.

Said *particle size* is possibly obtainable through specific sieving and sorting of miniparticles after the passage V, through sieves/vibrating sieves with appropriate nets.

Steps of sieving and sorting of only partially coated miniparticles may also be present after some steps of the process disclosed above, for example after the steps II, III and IV. Said passages of sieving and sorting are similar to step I. of the above mentioned process, possibly changing only the size of the net of sieves/vibrating sieves used.

It is therefore an aspect of the present invention a process as just disclosed above wherein after each of the steps II and/or III and/or IV and/or V there is a passage of sieving and sorting of the obtained material.

In the miniparticle in accordance to the present invention it is nevertheless obtained a progressive specific profile of release of ferrous fumarate, folic acid and vitamin C.

In processes for the preparation of miniparticles the release rate of the polymer layers/films disclosed in the related process steps, corresponds to that of the layers of polymers/film specifically described for the miniparticles.

It is a further object of the present invention an optionally polymeric matrix or of other type suitable for use in the pharmaceutical field or in the field of supplements, comprising miniparticles as defined above. Said matrix is intended for the release of miniparticles and acts as a container of the same.

Said matrix is very useful as it is not affected by problems of tabletting and by phenomena of surface polymerization, typical of active substances with low melting point, such as for example the thioctic acid, with consequent block of the release and therefore changing of the delayed or control effect .

The miniparticles disclosed in accordance with the present invention can be used as such or be mixed with excipients such as diluents, disgregants, lubricants, sweetening and flavoring agents depending on the form of administration and final user (see paediatric use and the use of flavoring agents in the dosage forms).

It is a further object of the present invention a formulation for oral use selected in the group comprising a sachet, a tablet, a capsule, a granulate, a vial, an extemporaneous suspension which contains a set of multilayered miniparticles as defined above optionally prepared by the processes listed above.

Where it is not specified or otherwise specified, the percentages in the text are intended as w/w percentages; while for the release rate they are intended as mg/h percentages.

Here below illustrative and not limitative examples of the present invention are reported.

### Example 1: PRODUCTION PROCESS OF MINIPARTICLES with thioctic acid with fast and slow release (FAST + retard)

*Phase 1 - Sorting the raw material of thioctic acid having a particle size between 188 µm and 630 µm*.
1) Fit on the vibrating sieve "WESTON" a net of 630 µm, place below the mouth of the same a suitable container for collecting the selected product.

Press the start button and proceed with the sorting of the raw material, putting on the net about 1.0 kg of thioctic acid, by slightly pressing, in a rotational direction, with the hand provided with a glove.

When the entire fraction of thioctic acid with *particle size* < 630 µm is passed in the lower part of the net, remove the fraction of thioctic acid with *particle size >* 630 µm and put it in an identified container.

Repeat the previous step until reaching the amount necessary for the production of the batch of thioctic acid with *particle size of* less than 630 µm (about 3.50 kg).

2) Remove from the vibrating sieve the net of 630 µm and install the net of 188 µm, place below the mouth of the same a suitable container for the collection of the selected product.

Press the start button and proceed with the sorting of the raw material, putting on the net about 1.0 kg of thioctic acid having *particle size* lower than 630 µm, by slightly pressing, in a rotational direction, with the hand provided with a glove.

When the entire fraction of thioctic acid < 188 µm is passed in the lower part of the net, remove the product remaining on the net and put it in a suitable container for storage.

Repeat the previous operation until reaching the amount necessary for the production of the batch (3.00 kg) of thioctic acid having a *particle size* between 188 and 630 µm.

Set aside the fraction < 188 µm.

### Phase 2 - sieving raw material of thioctic acid having particle size < 80 µm

Fit onto the vibrating sieve "WESTON" a net of 80 µm, place below the mouth of the same a suitable container for the collection of the sieved product.

Press the start button and proceed with the sieving of the raw material, putting on the net about 1.0 kg of thioctic acid with *particle size* < 250 µm, by slightly pressing, in a rotational direction, with the hand provided with a glove.

Remove the fraction of thioctic acid with *particle size >* 80 µm and put it in an identified container.

Repeat the previous operation until reaching the quantity necessary for the production of the batch (1.50 kg) of thioctic acid with *particle size* lower than 80 µm.

*Phase 3 - Preparation of the solution of 5% w*/*w ethylcellulose in* 96% *ethanol.* Introduce in a glass beaker with a capacity of about 5x10⁻³ m³ (liters), 2.375 kg of 96% ethanol. Add slowly under stirring 0.125 kg of ethylcellulose, kept under stirring until complete dissolution, about 4 x3600 s (hours).

*Phase 4 - Preparation of the solution of 20% w*/*w shellac in* 96% *ethanol.* Introduce in a glass beaker with a capacity of about 2 x10⁻³ m³ (liters), 0.800 kg of 96% ethanol. Add slowly under stirring 0.200 kg of shellac, kept under stirring until complete dissolution, about 2 x3600 s (hours).

*Phase 5 - Adjustment of the flow rate of the solution of 5% w*/*w ethylcellulose in ethanol 96%.*

Introducing into the container containing the solution of 5% w/w ethylcellulose the tube connected to the peristaltic pump.

Set the speed of the peristaltic pump at a speed of 5 s⁻¹ (rpm).

Place near the nozzle of the atomizer a suitable container for the collection of the solution.

Press the start button of the peristaltic pump and wait until the flow is constant. Calibrate the container and carry out the test of delivery for 60 s (minute). (theoretical amount 30 g, limits 28 g - 32 g)
**If** the amount delivered is less than the limit, increase the revolutions of the pump, if the amount is greater decrease them.

At the end of the tests, recover the solution putting it in the container.

*Phase 6 - Coating of the thioctic acid with 1.00 kg of solution of 5% w*/*w ethylcellulose in* 96% *ethanol and 0.900 kg of talc.*

Put in the coating pan with a capacity of about 10 x10⁻³ m³ (liters), 3.00 kg of the fraction of thioctic acid with *particle size* comprised between 188 and 630 µm of thioctic acid raw material previously selected, in accordance with *phase 1.*

Put in rotation the coating pan at a speed of 30 s⁻¹ (rpm) ± 1 s⁻¹ (rpm), add 30 g of talc and rotate for 5 x60 s (minutes) before starting the coating.

Place the atomizer at about 15 cm from the rotating mass and directing the jet toward the left upper quadrant, adjust the atomising pressure of the atomizer to 0.4 bar.

Spray 20 g of solution of 5% w/w ethylcellulose in 96% ethanol in about 40 seconds, at the end manually sprinkle 18 g of talc.

Wait approximately 2 x60 s (minutes) before repeating the previous operation, continue in the same way until on the thioctic acid with *particle size* comprised between 188 and 630 µm is put 1.00 kg of solution and 0.900 kg of talc.

At the end, let it dry in the coating pan at ambient temperature the thioctic acid coated with 5% ethylcellulose , at a speed of 15 s⁻¹ (rpm) ± 1 s⁻¹ (rpm) for at least 5 x3600 s (hours).

A sample to be sent to the QC (Quality Control) for the determination of the assay and in vitro releases.

Please note: the amounts of solution of 5% ethylcellulose and talc of the subsequent coating steps are decided on the basis of the analytical results obtained.

*Phase 7* - *Sorting the product thioctic acid coated with 5% ethylcellulose obtained in phase 6.*
1) Fit on the vibrating sieve "WESTON" a net of 790 µm, place below the mouth of the same a suitable container for the collection for the selected product.

Press the start button and proceed with the sorting, putting on the net about 1.0 kg of thioctic acid coated with 5% ethylcellulose obtained in *phase* 6, pressing slightly, in a rotational direction, with the hand provided with a glove.

When the entire fraction with *particle size* < 790 µm is passed in the lower part of the net, remove the fraction with *particle size >* 790 µm and put it in a container identified as "waste".

Repeat the previous operation until the end of the sorting of thioctic acid coated with 5%ethylcellulose and having *particle size* lower than 790 µm.

2) Remove from the vibrating sieve the net of 790 µm and install the net of 188 µm, place below the mouth of the same a suitable container for the collection of the selected product.

Press the start button and proceed with the sorting of the product, by putting on the net about 1.0 kg of thioctic acid coated with 5% ethylcellulose having *particle size* lower than 790 µm, by slightly pressing, in a rotational direction, with the hand provided with a glove.

When the entire fraction having a *particle size* < 188 µm is passed in the lower part of the net, remove the product remained on the net of thioctic acid coated with 5% ethylcellulose having *particle size* ranging between 188 and 790 µm and put it back into the coating pan.

Repeat the previous operation until the end of sorting.

Discard the fraction of thioctic acid coated with 5% ethylcellulose having *particle size* < 188 µm.

*Phase 8 - Adjustment of the flow rate of the solution of 20% w*/*w shellac in 96% ethanol.*

Introduce into the container containing the solution of 20% shellac the tube connected to the peristaltic pump.

Set the speed of the peristaltic pump at a speed of 5 s⁻¹ (rpm).

Place near the nozzle of the atomizer a suitable container for the collection of the solution.

Turn on the peristaltic pump and wait until the flow rate of the pump is constant. Calibrate the container and carry out the test of delivery for 60 s (minute). (Theoretical amount 30 g, limits 28 g - 32 g)

If the amount delivered is less than the limit, increase the revolutions of the pump, if it is greater decrease them.

At the end of the tests recover the solution putting it into the container.

*Phase 9 - Application of the raw material of thioctic acid < 80 µm to the product as obtained in phase 7.*

Put in rotation the coating pan at a speed of 30 s⁻¹ (rpm) ± 1 s⁻¹ (rpm).

Place the atomizer at about 15 cm from the rotating mass and direct the jet toward the left upper quadrant, adjust the atomising pressure of the atomizer at 1.0 bar. Spray 16 g of solution of 20% w/w shellac in 96% ethanol in about 32 seconds, at the end manually sprinkle 30 g of thioctic acid with *particle size* < 80 µm.

Wait approximately 2 x60 s (minutes) before repeating the previous operation, continue in the same way until it is applied on the product, as obtained in *phase 7,* 0.800 kg of solution of 20% w/w shellac and 1.50 kg of thioctic acid with *particle size* < 80 µm. At the end, dry the product obtained in *phase 7* now coated with 20% w/w shellac and thioctic acid with *particle size* < 80 µm in the coating pan at ambient temperature, at a speed of 15 s⁻¹ (rpm) ± 1 s⁻¹ (rpm) for at least 5 x3600s (hours).

*Phase 10 - Sorting the product* as *obtained in phase 9.*
1) Fit on the vibrating sieve "WESTON" a net of 850 µm, place below the mouth of the same a suitable container for the collection for the selected product.

Press the start button and proceed with the sorting, putting on the net about 1.0 kg of product as obtained in *phase 9,* pressing slightly, in a rotational direction, with the hand provided with a glove.

When the entire fraction with *particle size* < 850 µm is passed in the lower part of the net, remove the fraction with *particle size >* 850 µm and put it in a container identified as "waste".

Repeat the previous operation until the end of sorting of the product obtained with *particle size* < 850 µm.

2) Remove from the vibrating sieve the net of 850 µm and install the net of 210 µm, place below the mouth of the same a suitable container for the collection of the selected product.

Press the start button and proceed with the sorting of the product, by putting on the net about 1.0 kg of product as obtained at point 1) of said *phase 10* with *particle size* < 850 µm, by slightly pressing, in a rotational direction, with the hand provided with a glove.

When the entire fraction having a particle size < 210 µm is passed in the lower part of the net, remove the product remaining on the net having a *particle size* between 210 µm and 850 µm and put it back into the coating pan.

Repeat the previous operation until the end of sorting.

Discard the fraction having *particle size* < 210 µm.

*Phase 11 - Coating of the product obtained in phase 10 with 0.600 kg of solution of 5% w*/*w ethylcellulose in 96% ethanol and 0.600 kg of talc.*

Put in rotation the coating pan at a speed of 30 s⁻¹ (rpm).

Place the atomizer at about 15 cm from the rotating mass and direct the jet toward the upper quadrant on the left, adjust the atomising pressure of the atomizer to 0.4 bar.

Spray 20 g of solution of 5% w/w ethylcellulose in 96% ethanol in about 40 seconds, at the end manually sprinkle 20 g of talc.

Wait approximately 2 x60 s (minutes) before repeating the previous operation, continue in the same way until it is applied on the product obtained in *phase* 10 having a *particle size* between 210 µm and 850 µm, 0.600 kg of solution and 0.600 kg of talc.

At the end, dry the product thus obtained in the coating pan at ambient temperature, at a speed of 15 s⁻¹ (rpm) ± 1 s⁻¹ (rpm) for at least 5 x3600 s (hours).

*Phase 12 - Final sorting the product obtained in phase 11.*

Put in rotation the coating pan at a speed of 15 s⁻¹ (rpm) ± 1 s⁻¹ (rpm), add 5 g of Aerosil and rotate for 5 x60s (minutes).
1) Fit on the vibrating sieve "WESTON" a net of 850 µm, place below the mouth of the same a suitable container for the collection of the selected product.

Press the start button and proceed with the sorting putting on the net about 1.0 kg of product as obtained in *phase 11,* by slightly pressing, in a rotational direction, with the hand provided with a glove.

When the entire fraction having a *particle size* < 850 µm is passed in the lower part of the net, remove the fraction having *particle size >* 850 µm and put it in a container identified as "waste".

Repeat the previous operation until the end of the sorting of the obtained product having a *particle size* < 850 µm.

2) Remove from the vibrating sieve the net of 850 µm and install the net of 210 µm, place below the mouth of the same a suitable container for the collection for the selected product.

Press the start button and proceed with the sorting of the product, by putting on the net about 1.0 kg of product obtained as in point 1) of said *phase 12,* pressing slightly, in a rotational direction, with the hand provided with a glove.

When the entire fraction having a *particle size* < 210 µm is passed in the lower part of the net, remove the product remaining on the net having *particle size* ranging between 210 and 850 µm and put it in the container provided for packaging. Repeat the previous operation until the end of sorting.

Discard the fraction having *particle size* < 210 µm.

A sample to be sent to the Quality Control (QC) for the determination of the assay and in vitro releases, as from the following *phase 13.*

*Phase 13: Determination of the assay and in vitro releases.*
Instrumentation: Dissolution test
Method: USP
Parameters: 1000 x0,001 m^3 ml) buffer pH 6.5 E. Ph. 75 s⁻¹ (rpm) apparatus 2 (paddle) Nominal assay in thioctic acid: 671.1 x0,001 kg/kg (mg/g). - Actual assay: 670.7 x0,001 kg/kg (mg/g)

| Dissolution | Objective | Result |
|---|---|---|
| 1 x3600s. | 40-60% | 48.1% |
| 4 x3600s. | 60-80% | 73.9 % |
| 8 x3600s. | >= 85% | 98.0% |

Comparative

### Example 2: PRODUCTION PROCESS OF MINIPARTICLE with caffeine with fast and slow release (FAST + retard)

*Phase 1 - Sorting the raw material of caffeine having particle size ranging between 250 and 790 µm*
1) Fit on the vibrating sieve "WESTON" a net of 250 µm, place below the mouth of the same a suitable container for collecting the selected product.

Press the Start button and proceed with the sorting of the raw material, putting on the net about 1.0 kg of caffeine, slightly pressing, in a rotational direction, with the hand provided with a glove.

When the entire fraction of caffeine with particle size < 790 µm is passed in the lower part of the net, remove the fraction of caffeine with particle size > 790 µm and put it in an identified container.

Repeat the previous step until reaching the amount necessary for the production of the batch of caffeine with *particle size of* less than 790 µm (about 3.50 kg).

2) Remove from the vibrating sieve the net of 790 µm and install the net of 250 µm, place below the mouth of the same a suitable container for the collection for the selected product.

Press the start button and proceed with the sorting of the raw material, putting on the net about 1.0 kg of caffeine having *particle size* lower than 790 µm, by slightly pressing, in a rotational direction, with the hand provided with a glove.

When the entire fraction of caffeine < 250 µm is passed in the lower part of the net, remove the product which remains on the net and put it in a suitable container for storage.

Repeat the previous operation until reaching the amount necessary for the production of the batch (3.50 kg) of caffeine having *particle size* ranging between 250 and 790 µm.

Set aside the fraction < 250 µm.

### Phase 2 - sieving raw material of caffeine having particle size < 80 µm

Fit onto the vibrating sieve "WESTON" a net of 80 µm, place below the mouth of the same a suitable container for the collection of the sieved product.

Press the start button and proceed with the sieving of the raw material, putting on the net about 1.0 kg of caffeine with particle size < 250 µm, by slightly pressing, in a rotational direction, with the hand provided with a glove.

Remove the fraction of caffeine with *particle size >* 80 µm and put it in an identified container.

Repeat the previous operation until it is reached the amount necessary for the production of the batch (0.70 kg) of caffeine with *particle size <* 80 µm.

*Phase 3 - Preparation of the solution of shellac and 20% w*/*w hydroxypropylcellulose in* 96% *ethanol.*

Introduce in a glass beaker with a capacity of about 5 x10⁻³ m³ (liters), 1.600 kg of 96% ethanol. Add slowly under stirring 0.080 kg of hydroxypropylcellulose and leave under stirring until complete dissolution (about 4 x3600 s (hours)).

Slowly add 0.320 kg of shellac, keep under stirring until complete dissolution, about 2 x3600 s (hours).

*Phase 4 - Preparation of the solution of 30% w*/*w shellac in* 96% *ethanol.* Introduce in a glass beaker with a capacity of about 2 x10⁻³ m³ (liters), 0.700 kg of 96% ethanol. Add slowly under stirring 0.300 kg of shellac, keep under stirring until complete dissolution, about 2 x3600 s (hours).

*Phase 5 - Adjustment of the flow rate of the solution of shellac and 20% w*/*w hydroxypropylcellulose in 96% ethanol.*

Introduce into the container containing the solution of shellac and 20% w/w hydroxypropylcellulose in 96% ethanol the tube connected to the peristaltic pump. Set the speed of the peristaltic pump at a speed of 5 s⁻¹ (rpm) .

Place near the nozzle of the atomizer a suitable container for the collection of the solution.

Press the start button of the peristaltic pump and wait until the flow is constant. Calibrate the container and carry out the test of delivery for 60 s (minute). (Theoretical amount 20 g, limits 18 g - 22 g).

If the amount delivered is less than the limit, increase the revolutions of the pump, if is greater decrease them.

At the end of the tests recover the solution putting it in the container.

*Phase 6 - Coating of the caffeine with 1.00 kg of shellac and 20% w*/*w hydroxypropylcellulose in 96% ethanol and 0.300 kg of talc.*

Put in the coating pan with a capacity of about 10 x10⁻³ m³ (liters), 3.50 kg of the fraction of caffeine with particle size comprised between 250-790 µm of caffeine raw material previously selected, in accordance with phase 1.

Put in rotation the coating pan at a speed of 35 s⁻¹ (rpm) ± 1 s⁻¹ (rpm).

Place the atomizer at about 15 cm from the rotating mass and directing the jet toward the left upper quadrant, adjust the atomising pressure of the atomizer to 0.3 bar.

Spray 20 g of shellac and 20% w/w hydroxypropylcellulose in 96%ethanol in about 40 seconds, at the end manually sprinkle 6.6 g of talc.

Wait approximately 2 x60 s (minutes) before repeating the previous operation, continue in the same way until it is applied on the caffeine with particle size comprised between 250 and 790 µm 1.00 kg of solution and 0.300 kg of talc. At the end, let dry the caffeine in the coating pan at ambient temperature coated with shellac and 20% w/w hydroxypropylcellulose in 96% ethanol, at a speed of 15 s⁻¹(rpm) ± 1 s⁻¹ (rpm) for at least 5 x3600 s (hours).

A sample to be sent to the Quality Control (QC) for the determination of the assay and in vitro releases.

Please note: the amounts of solution of shellac and 20% w/w hydroxypropylcellulose in 96% ethanol and talc of the subsequent steps of coating are decided on the basis of the analytical results obtained.

*Phase 7 - Sorting the product caffeine coated with shellac and 20% w*/*w hydroxypropylcellulose in 96% ethanol obtained in phase 6.*
1) Fit on the vibrating sieve "WESTON" a net of 790 µm, place below the mouth of the same a suitable container for collecting the selected product.

Press the start button and proceed with the sorting, putting on the net about 1.0 kg of caffeine coated with shellac and 20% w/w hydroxypropylcellulose in 96% ethanol obtained in *phase 6,* by slightly pressing, in a rotational direction, with the hand provided with a glove.

When the entire fraction with *particle size* < 790 µm is passed in the lower part of the net, remove the fraction with *particle size >* 790 µm and put it in a container identified as "waste".

Repeat the previous operation until the end of the sorting of caffeine coated with shellac and 20% w/w hydroxypropylcellulose in 96% ethanol having *particle size <* 790 µm.

2) Remove from the vibrating sieve the net of 790 µm and install the net of 250 µm, place below the mouth of the same a suitable container for collecting the product selected.

Press the start button and proceed with the sorting of the product, by putting on the net about 1.0 kg of caffeine coated with shellac and 20% w/w hydroxypropylcellulose in 96% ethanol having *particle size* lower than 790 µm, by slightly pressing, in a rotational direction, with the hand provided with a glove.

When the entire fraction having a *particle size* < 250 µm is passed in the lower part of the net, remove the product remaining on the net of caffeine coated with shellac and 20% w/w hydroxypropylcellulose in 96% ethanol having a *particle size* between 250 and 790 µm and put it back into the coating pan.

Repeat the previous operation until the end of sorting.

Discard the fraction of the obtained product having a *particle size* < 250 µm.

*Phase 8 - Adjustment of the flow rate of the solution of 30% w*/*w shellac in 96% ethanol.*

Introduce into the container containing the solution of 30% shellac the tube connected to the peristaltic pump.

Set the speed of the peristaltic pump at a speed of 5 s⁻¹ (rpm)

Place near the nozzle of the atomizer a suitable container for the collection of the solution.

Turn on the peristaltic pump and wait until the flow rate of the pump is constant. Calibrate the container and carry out the test of delivery for 60 s (minute).

(Theoretical amount 20 g, limits 18 g - 22 g)

If the quantity delivered is less than the limit, increase the revolutions of the pump, if it is greater decrease them.

At the end of the tests, recover the solution putting it in the container.

*Phase 9 - Application of the raw material of Caffeine < 80 µm to the product* as *obtained in phase 7.*

Put in rotation the coating pan at a speed of 30 s⁻¹ (rpm) ± 1 s⁻¹ (rpm).

Introduce into the coating pan 2.64 Kg of caffeine retard obtained from phase 7.

Place the atomizer at about 15 cm from the rotating mass and directing the jet toward the left upper quadrant, adjust the atomising pressure of the atomizer to 0.3 bar.

Spray 26 g of solution of 30% w/w shellac in 96% ethanol in about 20 seconds, at the end manually sprinkle 13 g of caffeine with particle size < 80 µm.

Wait approximately 2 x60 s (minutes) before repeating the previous operation, continue in the same way until it is applied on the product, as obtained in phase 7, 1.313 kg of solution of 30% w/w shellac and 0.70 kg of caffeine with particle size < 80 µm.

At the end, dry the product obtained in phase 7 now coated with 30% w/w shellac and caffeine with particle size < 80 µm in the coating pan at ambient temperature, at a speed of 15 s⁻¹ (rpm) ± 1 s⁻¹ (rpm) for at least 6 x3600 s (hours).

*Phase 10 - Sorting the product* as *obtained in phase 9.*
1) Fit on the vibrating sieve "WESTON" a net of 850 µm, place below the mouth of the same a suitable container for the collection of the selected product.

Press the start button and proceed with the sorting, putting on the net about 1.0 kg of product as obtained in phase 9, by slightly pressing, in a rotational direction, with the hand provided with a glove.

When the entire fraction with *particle size* < 850 µm is passed in the lower part of the net, remove the fraction with *particle size >* 850 µm and put it in a container identified as "waste".

Repeat the previous operation until the end of sorting the product obtained with *particle size* < 850 µm.

Remove from the vibrating sieve the net of 850 µm and install the net of 210 µm, place below the mouth of the same a suitable container for the collection for the selected product.

2) Press the start button and proceed with the sorting of the product, by putting on the net about 1.0 kg of product as obtained in point 1) of said phase 10 with *particle size* < 850 µm, by slightly pressing, in a rotational direction, with the hand provided with a glove.

When the entire fraction having a *particle size* < 210 µm is passed in the lower part of the net, remove the product remaining on the net having a *particle size* between 210 µm and 850 µm and put it back into the coating pan.

Repeat the previous operation until the end of sorting.

Discard the fraction having *particle size* < 210 µm.

*Phase 11 - Coating of the product obtained in phase 10 with 0.300 kg* of shellac and 20% w/w hydroxypropylcellulose in 96% ethanol and 0.100 kg of talc. Put in rotation the coating pan at a speed of 30 s⁻¹ (rpm).

Introduce into the coating pan all the amount obtained from *phase 10.*

Place the atomizer at about 15 cm from the rotating mass and direct the jet toward the left upper quadrant, adjust the atomising pressure of the atomizer to 0.3 bar. Spray 20 g of solution of shellac and 20% w/w hydroxypropylcellulose in 96% ethanol in about 40 seconds, at the end manually sprinkle 7 g of talc.

Wait approximately 2 x60 s (minutes) before repeating the previous operation, continue in the same way until it is applied on the product obtained in *phase 10,* having a *particle size* between 210 µm and 850 µm, 0.300 kg of solution and 0.100 kg of talc.

At the end, dry the product thus obtained in the coating pan at ambient temperature, at a speed of 15 s⁻¹ (rpm) ± 1 s⁻¹ (rpm) for at least 6 x3600 s (hours).

*Phase 12 - Sorting the final product obtained in phase 11.*

Put in rotation the coating pan at a speed of 15 s⁻¹ (rpm) ± 1 s⁻¹ (rpm), add 5 g of Aerosil and rotate for 5 x60 s (minutes).
1) Fit on the vibrating sieve "WESTON" a net of 850 µm, place below the mouth of the same a suitable container for the collection for the selected product.

Press the start button and proceed with the sorting putting on the net about 1.0 kg of product as obtained in phase 11, by slightly pressing, in a rotational direction, with the hand provided with a glove.

When the entire fraction having a *particle size* < 850 µm is passed in the lower part of the net, remove the fraction having *particle size >* 850 µm and put it in a container identified as "waste".

Repeat the previous operation until the end of the sorting of the obtained product having a *particle size* < 850 µm.

2) Remove from the vibrating sieve the net of 850 µm and install the net of 210 µm, place below the mouth of the same a suitable container for the collection for the selected product.

Press the start button and proceed with the sorting of the product, putting on the net about 1.0 kg of product obtained as in point 1) of said *phase* 12, pressing slightly, in a rotational direction, with the hand provided with a glove.

When the entire fraction having a *particle size* < 210 µm is passed in the lower part of the net, remove the product remaining on the net having *particle size* ranging between 210 and 850 µm and put it in the container provided for packaging. Repeat the previous operation until the end of the sorting.

Discard the fraction having *particle size* < 210 µm.

A sample to be sent to the Quality Control (QC) for the determination of the assay and in vitro releases.

*Phase 13: Determination of the assay and the releases in vitro* Instrumentation: Dissolution test
UHPLC - PDA
Method: E.Ph./USP
Parameters: 900 x0,001 m^3 (ml) HCl 0 N; 75 s⁻¹ (rpm), apparatus 2 (paddle)
Nominal assay in caffeine: 561.3 x0,001 kg/kg (mg/g) - analytical assay in caffeine 568.6 x0,001 kg/kg (mg/g)

| Dissolution | Objective | Result |
|---|---|---|
| 1h | 20-40% | 32.6% |
| 4h | 40-60% | 51.2% |
| 8h | ≥ 65% | 70.3% |

### Example 3: PRODUCTION PROCESS OF MINIPARTICLE WITH VITAMIN C, ferrous fumarate and folic acid.

*Phase 1 - Sorting the raw material of Vitamin C comprised between 630 and 250 µm*
1) Fit on the vibrating sieve "WESTON" a net of 630 µm, place below the mouth of the same a suitable container for collecting the selected product.

Press the Start button and proceed with the sorting of the raw material, putting on the net about 1.0 kg of vitamin C, by slightly pressing, in a rotational direction, with the hand provided with a glove.

When the entire fraction < 630 µm is passed in the lower part of the net, remove the fraction > 630 µm and put it in an identified container.

Repeat the previous operation until it is reached the amount necessary for the production of the batch of vitamin C with *particle size of* less than 630 µm (about 3.50 kg).

2) Remove from the vibrating sieve the net of 630 µm and install the net of 250 µm, place below the mouth of the same a suitable container for the collection of the selected product.

Press the start button and proceed with the sorting of the raw material, putting on the net about 1.0 kg of vitamin C with *particle size of* less than 630 µm, by slightly pressing, in a rotational direction, with the hand provided with a glove.

When the entire fraction of vitamin C with *particle size* < 250 µm is passed in the lower part of the net, remove the product remaining on the net and put it in a suitable container for storage.

Repeat the previous operation until it is reached the amount necessary for the production of the batch (3,00 kg) of Vitamin C with *particle size* comprised between 250 µm and 630 µm.

Set aside the fraction < 250 µm.

*Phase 2 - Preparation of the hydroalcoholic solution of 5% w*/*w HPMC in 96% ethanol* / *filtered H₂O.*

Introduce in a glass beaker with a capacity of about 1 x10⁻³ m³ (liter), 0.380 kg of 96% ethanol and 0.095 kg of filtered H₂O.

Add slowly under stirring 0.025 kg of HPMC, keep under stirring until complete dissolution, about 4 x3600 s (hours).

*Phase 3 - Preparation of the solution of 20% w*/*w shellac in* 96% *ethanol.* Introduce in a glass beaker with a capacity of about 5 x10⁻³ m³ (liters), 2.400 kg of 96% ethanol. Add slowly under stirring 0.600 kg of shellac, kept under stirring until complete dissolution, about 2 x3600 s (hours).

*Phase 4 - Preparation of the solution of shellac + 20% w*/*w hydroxypropyl cellulose in* 96% *ethanol.*

Introduce in a glass beaker with a capacity of about 5 x10⁻³ m³ (liters), 1.600 kg of 96% ethanol.

Add slowly under stirring 0.080 kg of hydroxypropylcellulose and leave under stirring until complete dissolution (about 4 x3600 s (hours)).

Slowly add 0.320 kg of shellac, keep under stirring until complete dissolution, about 2 x3600 s (hours).

*Phase 5 - Preparation of the hydroalcoholic solution of 2.5% w*/*w folic acid in 96% ethanol* / *filtered H₂O.*

Introduce in a glass beaker with a capacity of about 500 x0,001 m^3 (ml), 0.150 kg of filtered H₂O, add 7.7 g of folic acid and put under stirring until complete dissolution (about 30 x60 s (minutes)).

Add 0.150 kg of 96% ethanol.

*Phase 6 - Adjustment of the flow rate of the hydroalcoholic solution of 5% w*/*w HPMC in 96% ethanol* / *filtered H₂O.*

Introduce into the container containing the solution of 5% HPMC the tube connected to the peristaltic pump.

Set the peristaltic pump at a speed of 5 s⁻¹ (rpm).

Place in the vicinity of the nozzle of the atomizer a suitable container for the collection of the solution.

Press the start button of the peristaltic pump and wait until the flow is constant.

Calibrate the container and carry out the test of delivery for60 s (minute).

(Theoretical amount 25 g, limits 23 g - 27 g)

If the amount delivered is less than the limit, increase the revolutions of the pump, if it is greater decrease them.

At the end of the tests to recover the solution, putting it in the container.

*Phase 7 - Coating of vitamin C with 0.500 kg of the hydroalcoholic solution of 5% w*/*w HPMC in 96% ethanol*/ *filtered H₂O and 0.200 kg of talc.*

Put in the coating pan with a capacity of about 10 x10⁻³ m³ (liters), 3.00 kg of fraction comprised between 250 and 630 µm of Vitamin C raw material previously selected in *phase 1* in said example 3.

Put in rotation the coating pan at a speed of 30 s⁻¹ (rpm) ± 1 s⁻¹ (rpm).

Place the atomizer at about 15 cm from the rotating mass and direct the jet toward the left upper quadrant, adjust the atomising pressure of the atomizer to 0.6 bar. Spray 10 g of solution of 5% w/w HPMC in 96% ethanol/filtered H₂O in about 24 seconds, at the end manually sprinkle 4 g of talc.

Wait approximately 2 x60 s (minutes) before repeating the previous operation, continue in the same way until it is applied 0.500 kg of solution and 0.200 kg of talc on vitamin C with *particle size* comprised between 250 and 630 µm.

At the end, dry Vitamin C thus coated with HPMC in the coating pan at ambient temperature, at a speed of 15 s⁻¹ (rpm) ± 1 s⁻¹ (rpm) for at least 3 x3600 s (hours).

*Phase 8 - Adjustment of the flow rate of the hydroalcoholic solution of 2.5% w*/*w folic acid in 96% ethanol*/ *filtered H₂O.*

Introduce into the container containing the hydroalcoholic solution of 2.5% w/w Folic Acid in 96%ethanol / filtered H₂O the tube connected to the peristaltic pump.

Set the peristaltic pump at a speed of 5 s⁻¹ (rpm).

Place near the nozzle of the atomizer a suitable container for the collection of the solution.

Press the start button of the peristaltic pump and wait until the flow is constant. Calibrate the container and carry out the test of delivery for60 s (minute).

(Theoretical amount 30 g, limits 28 g - 32 g).

If the amount delivered is less than the limit, increase the revolutions of the pump, if it is greater decrease them.

At the end of the tests recover the solution putting it again in the container.

### Step 9 - Application in continuous of the hydroalcoholic solution of 2.5% w/w folic acid in 96% ethanol/ filtered H₂O.

Introduce in the coating pan the product obtained from *phase 7* in said example 3. Put in rotation the coating pan at a speed of 30 s⁻¹ (rpm) ± 1 s⁻¹ (rpm).

Place the atomizer at about 15 cm from the rotating mass and direct the jet toward the left upper quadrant, adjust the atomising pressure of the atomizer to 0.4 bar. Spray the entire solution of 2.5% w/w of folic acid in continuous (307.7 g) with the peristaltic pump at a speed of 5 s⁻¹ (rpm) on the product in a coating pan.

At the end, dry Vitamin C coated with HPMC and folic acid in the coating pan at ambient temperature, at a speed of 15 s⁻¹ (rpm) ± 1 s⁻¹ (rpm) for at least 2 x3600 s (hours).

*Phase 10 - Adjustment of the flow rate of the solution of 20% w*/*w shellac in 96% ethanol.*

Introduce into the container containing the solution of 20% shellac the tube connected to the peristaltic pump.

Set the peristaltic pump at a speed of 5 s⁻¹ (rpm).

Place near the nozzle of the atomizer a suitable container for the collection of the solution.

Press the start button of the peristaltic pump and wait until the flow is constant. Calibrate the container and carry out the test of delivery for 60 s (minute).

(Theoretical amount 30 g, limits 28 g - 32 g).

If the amount delivered is less than the limit, increase the revolutions of the pump, if it is greater decrease them.

At the end of the tests to recover the solution putting it in the container.

*Phase 11 - Application of ferrous fumarate with the solution of 20% w*/*w shellac in 96% ethanol.*

Put in rotation the coating pan containing the product obtained in *phase 9* of said example 3 at a speed of 30 s⁻¹ (rpm) ± 1 s⁻¹ (rpm)

Place the atomizer at about 15 cm from the rotating mass and direct the jet toward the left upper quadrant, adjust the atomising pressure of the atomizer to 0.4 bar. Spray 26 g of solution of 20% w/w shellac in 96% ethanol in about 52 seconds, at the end manually sprinkle 20 g of ferrous fumarate.

Wait approximately 2 x60 s (minutes) before repeating the previous operation, continue in the same way until it is applied on the product of *phase 9* 2.280 kg of solution and 1.760 kg of ferrous fumarate.

At the end, allow the product to dry in the coating pan at ambient temperature, at a speed of 15 s⁻¹ (rpm) ± 1 s⁻¹ (rpm) for at least 5 x3600 s (hours).

*Phase 12 - Sorting of the product obtained.*
1) Fit on the vibrating sieve "WESTON" a net of 790 µm, place below the mouth of the same a suitable container for the collection of the selected product.

Press the start button and proceed with the sorting, putting on the net about 1.0 kg of product as obtained in *phase 11* of said example, slightly pressing, in a rotational direction, with the hand provided with a glove.

When the entire fraction < 790 µm is passed in the lower part of the net, remove the fraction > 790 µm and put it in a container identified as "waste".

Repeat the previous operation until the end of sorting of the obtained product with *particle size* < 790 µm.

2) Remove from the vibrating sieve the net of 790 µm and install the net of 250 µm, place below the mouth of the same a suitable container for the collection for the selected product.

Press the start button and proceed with the sorting, putting on the net about 1.0 kg of product obtained at point 1) of said *phase* 12, by slightly pressing, in a rotational direction, with the hand provided with a glove.

When the entire fraction < 250 µm is passed in the lower part of the net, remove the product remaining on the net and put it back into the coating pan.

Repeat the previous operation until the end of the sorting to obtain a product consisting of vitamin C as core coated with HPMC, folic acid and ferrous fumarate with *particle size* comprised between 250 and 790 µm.

Discard the fraction < 250 µm.

Weigh the product obtained and the wastes.

*Phase 13 - Adjustment of the flow rate of the solution of shellac + 20% w*/*w hydroxypropyl cellulose in 96% ethanol.*

Introduce into the container containing the solution of shellac + 20% w/w hydroxypropyl cellulose in 96% ethanol the tube connected to the peristaltic pump. Set the speed of the peristaltic pump at a speed of 5 s⁻¹ (rpm).

Place near the nozzle of the atomizer a suitable container for the collection of the solution.

Press the start button of the peristaltic pump and wait until the flow is constant. Calibrate the container and carry out the test of delivery for 60 s (minute).

(Theoretical amount 30 g, limits 28 g - 32 g).

**If** the amount delivered is less than the limit, increase the revolutions of the pump, if it is greater decrease them.

At the end of the tests recover the solution putting it in the container.

*Phase 14 - Coating of the product obtained with 1.300 kg of solution of shellac + 20% w*/*w hydroxypropyl cellulose in* 96% *ethanol and 1.000 kg of talc.*

Put in rotation the coating pan at a speed of 30 s⁻¹ (rpm).

Place the atomizer at about 15 cm from the rotating mass and directing the jet toward the left upper quadrant, adjust the atomising pressure of the atomizer at 0.5 bar.

Spray on the product obtained from *phase 12* of said example 3, 26 g of solution of shellac + 20% w/w hydroxypropyl cellulose in 96% ethanol in about 52 seconds, at the end manually sprinkle 20 g of talc.

Wait approximately 2 x60 s (minutes) before repeating the previous operation, continue in the same way until you have applied on the product 1.300 kg of solution and 1.000 kg of talc.

At the end, allow the product to dry in the coating pan at ambient temperature, at a speed of 15 s⁻¹ (rpm) ± 1 s⁻¹ (rpm) for at least 5 x3600 s (hours).

*Phase 15 - Sorting the final product obtained.*
1) Fit on the vibrating sieve "WESTON" a net of 790 µm, place below the mouth of the same a suitable container for the collection of the selected product.

Press the start button and proceed with the sorting, putting on the net about 1.0 kg of product as obtained in *phase 14,* by slightly pressing, in a rotational direction, with the hand provided with a glove.

When the entire fraction < 790 µm is passed into the lower part of the net, remove the fraction > 790 µm and put it in a container identified as "waste".

Repeat the previous operation until the end of sorting of the product with *particle size* < 790 µm.

2) Remove from the vibrating sieve the net of 790 µm and install the net of 250 µm, place below the mouth of the same a suitable container for the collection of the selected product.

Press the start button and proceed with the sorting, putting on the net about 1.0 kg of product as obtained in point 1) of said *phase 15,* by slightly pressing, in a rotational direction, with the hand provided with a glove.

When the entire fraction < 250 µm is passed in the lower part of the net, remove the product remaining on the net and put it in the container provided for packaging. Repeat the previous operation until the end of the sorting of final miniparticles with *particle size* between 250 and 790 µm.

Discard the fraction < 250 µm.

Weigh the product obtained and the wastes.

A sample to be sent to the Quality Control (QC) for comprehensive analysis in accordance with *phase 16* reported below.

*Phase 16: Determination of the assay and in vitro releases.*
Instrumentation: Dissolution test
ICP-AES

Parameters: 900 x0,001 m^3 (ml) acid pH E. Ph. 75 s⁻¹ (rpm) apparatus 2 (paddle)

| | |
|---|---|
| Assay expressed in Fe²⁺ | 85 x0,001 kg/kg (mg/g) + or - 10% |
| Assay in folic acid | 1.1 x0,001 kg/kg (mg/g) + or - 10% |
| Assay in vitamin C | 450 x0,001 kg/kg (mg/g) + or - 10% |
| Release of Fe2+ | |

| Dissolution | Objective | Result |
|---|---|---|
| 0.5 h | 20-40% | 30.1% |
| 1 h | 60-80% | 74.1% |
| 2 h | ≥ 85% | 100% |

## Claims

1. Multilayered miniparticle having a particle size ranging from 200 µm to 850 µm for oral administration comprising from inside to outside:
A) a core of an active substance;
B) an inner film layer which is protective or for the modified release;
C) a layer of at least an active substance which is equal to or different from the one in the core A);
D) an outer film layer which more rapidly releases compared to the inner film layer which is protective or for the modified release B), wherein the active substance is selected in the group comprising thioctic or alpha lipoic acid, iron or a salt thereof such as ferrous fumarate or bisglycinate, vitamin C, folic acid.

2. Multilayered miniparticle according to claim 1, wherein the core of active substance A) has a diameter of 188 µm - 630 µm in case the active substance is thioctic acid.

3. Multilayered miniparticle according to claims 1-3, wherein the inner film layer B) which is protective or for the modified release is made of a substance or polymer selected in the group comprising ethyl cellulose, hydroxypropyl methyl ethyl cellulose phthalate (HPMCP), hydroxypropyl methyl ethyl cellulose acetate succinate (HPMCAS), ethylene-vinyl acetate, a polyacrylic acid derivative, acrylic derivatives: copolymers of acrylic acid/methyl metacrylate (i.e. EUDRAGIT), shellac, hydroxypropylmethyl cellulose (HPMC) and hydroxypropyl cellulose (HPC).

4. Multilayered miniparticle according to claims 1-3, wherein the core A) and the layer C) are made of thioctic acid.

5. Multilayered miniparticle according to claims 1-4, wherein the outer film layer D) is made of a substance or polymer selected in the group comprising PCL-PVAc-PEG (i.e. Soluplus^{®}), hydroxypropylmethyl cellulose (HPMC), polyethylene glycol (PEG), hydroxyethyl cellulose (HEC), hydroxypropyl cellulose (HPC), polyvinyl alcohol (PVA), polyvinylpyrrolidone (PVP), shellac, ethyl cellulose and acrylic derivatives: copolymers of acrylic acid/methyl metacrylate (i.e. EUDRAGIT).

6. Process for preparing a multilayered miniparticle according to claims 1-5, comprising the following steps:
i. sieving and sorting an active substance having varied particle size to obtain particles of active substance with defined particle size;
ii. applying a solution of a polymer or film which is protective or for the modified release to each particle of active substance of step i. to obtain a core of active substance covered with a film layer which is protective or for the modified release;
iii. forming a layer of at least an active substance which is equal to or different from the one present in the core, on the covered core of active substance of step ii.;
iv. forming, on the layer formed at step iii, an outer film which releases more rapidly compared to the film layer which is protective or for the modified release of step ii.

7. Process according to claim 6, wherein after each of step ii., and/or iii. and/or iv. there is another sieving and sorting step of the obtained material.

8. Multilayered miniparticle according to claim 1 for oral administration comprising
a further layer (E) an outer film layer which releases more rapidly than the inner film layer which is protective or for the modified release b).

9. Multilayered miniparticle according to claim 8, wherein the core A) is of vitamin C, the layer C) is of folic acid, and the layer E) is of iron or a salt thereof such as ferrous fumarate or ferrous bisglycinate.

10. Process for preparing a miniparticle according to claim 7, wherein after steps i., ii. and iii. it comprises the steps of:
v. forming an outer film layer, on the layer formed at the previous step, which releases more rapidly compared to the film layer which is protective or for the modified release of step ii.

11. Process according to claim 10, wherein after each step ii. and/or iii. and/or iv. and/or v. there is a step of sieving or sorting of the obtained material.

12. Matrix comprising multilayered miniparticles according to claims 1-5, or 8-9.

13. Formulation for oral use selected in the group comprising a sachet, a tablet, a capsule, a granulate, a vial, an extemporaneous suspension, which contains a set of multilayered miniparticles as defined in claims 1-5 or 8-9.

## Patentansprüche

1. Mehrschichtige Mikropartikel mit einer Partikelgröße im Bereich von 200 µm bis 850 µm zur oralen Verabreichung, die von innen nach außen Folgendes umfasst:
A) einen Wirkstoffkern;
B) eine innere Filmschicht, die schützend wirkt oder der modifizierten Freisetzung dient;
C) eine Schicht aus zumindest einem Wirkstoff, der mit dem in Kern A) enthaltenen Wirkstoff identisch oder davon verschieden ist;
D) eine äußere Filmschicht, die im Vergleich zu der inneren Filmschicht B), welche schützend wirkt oder der modifizierten Freisetzung dient, eine schnellere Freisetzung bewirkt, wobei der Wirkstoff aus der Gruppe, die Thioctsäure oder Alpha-Liponsäure, Eisen oder ein Salz davon, wie beispielsweise Eisenfumarat oder Bisglycinat, Vitamin C oder Folsäure, umfasst, ausgewählt wird.

2. Mehrschichtige Mikropartikel nach Anspruch 1, wobei der Wirkstoffkern A) in dem Fall, dass der Wirkstoff Thioctsäure ist, einen Durchmesser von 188 µm bis 630 µm aufweist.

3. Mehrschichtige Mikropartikel nach den Ansprüchen 1-3, wobei die innere Filmschicht B), die schützend wirkt oder der modifizierten Freisetzung dient, aus einer Substanz oder einem Polymer besteht, die bzw. das aus der Gruppe, die Ethylcellulose, Hydroxypropylmethylethylcellulosephthalat (HPMCP), Hydroxypropylmethylethylcelluloseacetatsuccinat (HPMCAS), Ethylen-Vinylacetat, ein Polyacrylsäurederivat und Acrylatderivate umfasst, ausgewählt wird: nämlich Copolymere aus Acrylsäure/Methylmethacrylat (d.h. EUDRAGIT), Schellack, Hydroxypropylmethylcellulose (HPMC) und Hydroxypropylcellulose (HPC).

4. Mehrschichtige Mikropartikel nach den Ansprüchen 1-3, wobei der Kern A) und die Schicht C) aus Thioctsäure bestehen.

5. Mehrschichtige Mikropartikel nach den Ansprüchen 1-4, wobei die äußere Filmschicht D) aus einer Substanz oder einem Polymer besteht, die bzw. das aus der Gruppe, die PCL-PVAc-PEG (d.h. Soluplus^{®}), Hydroxypropylmethylcellulose (HPMC), Polyethylenglykol (PEG), Hydroxyethylcellulose (HEC), Hydroxypropylcellulose (HPC), Polyvinylalkohol (PVA), Polyvinylpyrrolidon (PVP), Schellack, Ethylcellulose und Acrylatderivate umfasst, ausgewählt wird: nämlich Copolymere aus Acrylsäure/Methylmethacrylat (d.h. EUDRAGIT).

6. Verfahren zur Herstellung einer mehrschichtigen Mikropartikel nach den Ansprüchen 1-5, das die folgenden Schritte umfasst:
i. Sieben und Klassieren eines Wirkstoffs mit variierender Partikelgröße, um Wirkstoffpartikel mit festgelegter Partikelgröße zu erhalten;
ii. Aufbringen einer Lösung eines Polymers oder eines Films, der schützend wirkt oder der modifizierten Freisetzung dient, auf jeden Wirkstoffpartikel aus Schritt i., um einen Wirkstoffkern zu erhalten, der mit einer Filmschicht überzogen ist, die schützend wirkt oder der modifizierten Freisetzung dient;
iii. Ausbilden einer Schicht aus zumindest einem Wirkstoff, der mit dem im Kern vorhandenen Wirkstoff identisch oder davon verschieden ist, auf dem beschichteten Wirkstoffkern aus Schritt ii.;
iv. Ausbilden eines äußeren Films auf der in Schritt iii. gebildeten Schicht, wobei dieser äußere Film im Vergleich zu der Filmschicht aus Schritt ii., die schützend wirkt oder der modifizierten Freisetzung dient, eine schnellere Freisetzung bewirkt.

7. Verfahren nach Anspruch 6, wobei nach jedem der Schritte ii. und/oder iii. und/oder iv. ein weiterer Schritt des Siebens oder Klassierens des erhaltenen Materials durchgeführt wird.

8. Mehrschichtige Mikropartikel nach Anspruch 1 zur oralen Verabreichung, die eine weitere Schicht (E) umfasst, nämlich eine äußere Filmschicht, die im Vergleich zu der inneren Filmschicht B), die schützend wirkt oder der modifizierten Freisetzung dient, eine schnellere Freisetzung bewirkt.

9. Mehrschichtige Mikropartikel nach Anspruch 8, wobei der Kern A) aus Vitamin C besteht, die Schicht C) aus Folsäure besteht und die Schicht E) aus Eisen oder einem Salz davon, wie beispielsweise Eisenfumarat oder Eisenbisglycinat, besteht.

10. Verfahren zur Herstellung eines Mikropartikels nach Anspruch 7, wobei nach den Schritten i., ii. und iii. die folgenden Schritte durchgeführt werden:
v. Ausbilden einer äußeren Filmschicht auf der in dem vorhergehenden Schritt gebildeten Schicht, wobei diese äußere Filmschicht im Vergleich zu der Filmschicht aus Schritt ii., die schützend wirkt oder der modifizierten Freisetzung dient, eine schnellere Freisetzung bewirkt.

11. Verfahren nach Anspruch 10, wobei nach jedem der Schritte ii. und/oder iii. und/oder iv. und/oder v. ein Schritt des Siebens oder Klassierens des erhaltenen Materials durchgeführt wird.

12. Matrix, die mehrschichtige Mikropartikel nach den Ansprüchen 1-5 oder 8-9 umfasst.

13. Darreichungsform zur oralen Anwendung, die aus der Gruppe, die ein Sachet, eine Tablette, eine Kapsel, ein Granulat, ein Fläschchen und eine extemporane Suspension umfasst, ausgewählt wird und einen Satz von mehrschichtigen Mikropartikeln enthält, wie in den Ansprüchen 1- 5 oder 8- 9 definiert.

## Revendications

1. Mini-particule multicouche ayant une taille de particule de 200 µm à 850 µm destinée à l'administration par voie orale comprenant de l'intérieur vers l'extérieur :
A) un noyau d'une substance active ;
B) une couche de film intérieure qui est de protection ou à libération modifiée ;
C) une couche d'au moins une substance active qui est égale ou différente de celle dans le noyau A) ;
D) une couche de film extérieure qui libère plus rapidement par rapport à la couche de film intérieure qui est de protection ou à libération modifiée B), dans laquelle la substance active est sélectionnée dans le groupe comprenant de l'acide thioctique ou alpha-lipoïque, du fer ou un sel de celui-ci, tel que du fumarate ou du bisglycinate ferreux, de la vitamine C, de l'acide folique.

2. Mini-particule multicouche selon la revendication 1, dans laquelle le noyau de la substance active A) présente un diamètre allant de 188 µm à 630 µm lorsque la substance active est de l'acide thioctique.

3. Mini-particule multicouche selon les revendications 1 à 3, dans laquelle la couche de film intérieure B) qui est de protection ou à libération modifiée est composée d'une substance ou d'un polymère sélectionné dans le groupe comprenant de l'éthylcellulose, du phtalate d'hydroxypropylméthyléthyl cellulose (HPMCP), de l'acétate-succinate d'hydroxypropylméthyléthyl cellulose (HPMCAS), de l'éthylène-acétate de vinyle, un dérivé de l'acide polyacrylique, des dérivés acryliques : des copolymères d'acide acrylique/méthacrylate de méthyle (c'est-à-dire EUDRAGIT), de la gomme-laque, de l'hydroxypropylméthylcellulose (HPMC) et de l'hydroxypropylcellulose (HPC).

4. Mini-particule multicouche selon les revendications 1 à 3, dans laquelle le noyau A) et la couche C) sont constitués d'acide thioctique.

5. Mini-particule multicouche selon les revendications 1 à 4, dans laquelle la couche de film extérieure D) est composée d'une substance ou d'un polymère sélectionné dans le groupe comprenant du PCL-PVAc-PEG (c'est-à-dire Soluplus^{®}), de l'hydroxypropylméthylcellulose (HPMC), du polyéthylène glycol (PEG), de l'hydroxyéthylcellulose (HEC), de l'hydroxypropylcellulose (HPC), de l'alcool polyvinylique (PVA), de la polyvinylpyrrolidone (PVP), de la gomme-laque, de l'éthylcellulose et des dérivés acryliques : des copolymères d'acide acrylique/méthacrylate de méthyle (c'est-à-dire EUDRAGIT).

6. Procédé de préparation d'une mini-particule multicouche selon les revendications 1 à 5, comprenant les étapes suivantes :
i. tamiser et classer une substance active ayant des tailles de particule variées pour obtenir des particules de substance active ayant une taille de particule définie ;
ii. appliquer une solution d'un polymère ou d'un film qui est de protection ou à libération modifiée sur chaque particule de substance active de l'étape i. afin d'obtenir un noyau de substance active enrobé d'une couche de film qui est de protection ou à libération modifiée ;
iii. former une couche d'au moins une substance active qui est égale ou différente de celle présente dans le noyau, sur le noyau enrobé de substance active de l'étape ii. ;
iv. former, sur la couche formée à l'étape iii, un film extérieur qui libère plus rapidement par rapport à la couche de film qui est de protection ou à libération modifiée de l'étape ii.

7. Procédé selon la revendication 6, dans lequel, après chaque étape ii., et/ou iii. et/ou iv., il y a une autre étape de tamisage et de classement du matériau obtenu.

8. Mini-particule multicouche selon la revendication 1 destinée à l'administration par voie orale comprenant
une couche supplémentaire (E), une couche de film extérieure qui libère plus rapidement que la couche de film intérieure qui est de protection ou à libération modifiée b).

9. Mini-particule multicouche selon la revendication 8, dans laquelle le noyau A) est de la vitamine C, la couche C) est de l'acide folique, et la couche E) est du fer ou un sel de celui-ci, tel que du fumarate ou du bisglycinate ferreux.

10. Procédé de préparation d'une mini-particule selon la revendication 7, dans lequel, après les étapes i., ii. et iii., il comprend les étapes consistant à :
v. former une couche de film extérieure, sur la couche formée lors de l'étape précédente, qui libère plus rapidement par rapport à la couche de film qui est de protection ou à libération modifiée de l'étape ii.

11. Procédé selon la revendication 10, dans lequel, après chaque étape ii. et/ou iii. et/ou iv. et/ou v., il y a une étape de tamisage ou de classement du matériau obtenu.

12. Matrice comprenant des mini-particules multicouches selon les revendications 1 à 5, ou 8 à 9.

13. Formulation destinée à l'utilisation orale sélectionnée dans le groupe comprenant un sachet, un comprimé, une capsule, un granulat, un flacon, une suspension extemporanée, qui contient un ensemble de mini-particules multicouches telles que définies dans les revendications 1 à 5 ou 8 à 9.
